# EUROPEAN PATENT APPLICATION

(11) **EP 1 609 454 A1**
(43) Date of publication of application: **28.12.2005**
(21) Application number: 05253653.9
(22) Date of filing: 14.06.2005
(51) Int. Cl.: A61J 9/08, A47J 41/00

(54) **Insulated food containers**

(30) Priority: 23.06.2004 US 875610
(71) Applicant: Clute, Lorne Jason, Alamo CA 94507 (US)
(72) Inventor: Lee, Sung, Sacramento California 95829 (US); McKendry, Bruce, Benicia California 94510 (US)
(74) Representative: Hitching, Peter Matthew

(57) **Abstract**

A container (10) for milk or the like includes an inner vessel (12) for containing the milk and an outer vessel (14) that creates a larger volume than the volume of the inner vessel (12), so that the inner vessel (12) can fit inside the outer vessel (14). In one embodiment, an insert (16) is placed between the top end (20) of the inner vessel (12) and the top end (24) of the outer vessel (14). The insert (16) has a plurality of threads (28) that engage threads on the inner vessel (12), and a cap (18) covers the top ends of the inner and outer vessels and the insert. The cap (18) has threads (32) that engage threads on the outer vessel (14). Hot or cold liquid can be placed in a cavity (34) formed between the inner and outer vessels. The cavity (34) is substantially sealed by the insert (16) to maintain, raise or lower the temperature of the milk in the inner vessel (12). In another embodiment, the insert (16) is not used.

## Description

This invention relates to insulated liquid and solid food containers, and more particularly, to human baby bottles, bottles for milk collection from breast pumps, and baby food containers, that tend to stabilize or increase the temperature of liquid and/or solid food in the containers.

It is well known that bottles are used for storing and feeding human milk, milk substitutes (such as infant formula) and other fluids. Bottles are also used as collection vessels for milk expressed from human breast pumps. These bottles commonly have a single wall construction, being made primarily of either plastic or glass.

It is preferred that infants, especially those considered at risk for starting life optimally in terms of nutritional intake, be fed milk at or very near their mother's body temperature. For improved growth and positive nutritional status, milk fed at or near body temperature is preferred.

Although it is preferable that milk fed to newborns be at or near body temperature, it is difficult to maintain this temperature under typical circumstances. Mothers expressing milk into single wall bottles can take more than 15 minutes to express. Even if this expressed milk is immediately taken to the baby for feeding, the temperature will have dropped several degrees while expressing, due to the poor insulating properties of the single wall bottle. As the expressed milk is fed to the baby, milk temperature continues dropping during the feeding session. Likewise, when other fluids are fed to the baby, fluid temperature continues to drop during the feeding event. The longer the baby takes to consume the fluids, the more the temperature can drop (assuming normal room temperature conditions). Therefore, there is a need for a baby bottle with improved insulating properties over typical, single wall bottles. Likewise, there is a need for containers that can be used for maintaining the temperature of solid baby food, as well.

Efforts have been made to provide double walled containers for milk and other fluids. For example, Diak/Ghanem U.S. Patent No. 6,631,819 discloses a double walled vessel that has a sealed cavity between the walls. This design results in a trapped air space between the two bottles. The air space acts as a thermal barrier that tends to retard temperature change relative to single walled bottles of like materials, but the air is permanently sealed in the space and will typically be at room temperature, not body temperature. Thus, there is a need for liquid and food containers that maintain their contents at or above body temperature in a better and more easily controlled manner.

There is also a need for an insulating collection vessel that offers convenience in use, and multiple use capability, such as offering an insulating benefit while expressing from a breast pump, and/or while feeding a baby from the bottle. A collection vessel, or combination of vessels, which adapts readily for both these, and other, purposes would be attractive to mothers and a benefit to babies.

Accordingly, it is desirable to provide new and improved insulated collection vessels.

It is also desirable to provide new and improved insulated collection vessels for human breast pumps.

It is desirable to provide new and improved insulated collection vessels for human breast pumps that can readily be used in feeding collected milk to the baby immediately upon expression of milk, thereby aiding in maintaining optimum milk temperatures through feeding.

It is desirable to provide new and improved insulated collection vessels for human milk substitute and/or stored or otherwise held human milk, which has been warmed to desired temperatures.

It is desirable is to provide means for adapting single wall containers to form a double wall container or milk vessel.

It is desirable to provide means for adapting single wall containers to form a double wall container that can easily and readily be used for the purpose of direct attachment to a breast pump for the collection of expressed milk, and also for the immediate acceptance of an artificial nipple, or other acceptable attachment such as a sippee cup spout, for immediate feeding.

It is desirable to provide in one embodiment an insulated bottle having a simple adapter that unites two typical baby bottles, selected for compatibility of purpose, which combination is convenient to use, and employs bottles which can be utilized individually as is common, thereby providing value to the user in both multiple function capability and economy.

It is desirable to provide an iteration of an insulated bottle assembly that includes the baby bottles, selected for compatibility of purpose, which combination is convenient to use, and employs bottles which can be utilized individually as is common, thereby providing value to the user via the economies inherent in multiple function capability. Further, an adapter part is not needed in this iteration, which reduces parts and complexity.

Embodiments of milk holding, feeding and/or collection bottle (vessel) apparatus are disclosed. In one embodiment, the apparatus has two vessels, a first outside vessel and a second inner vessel. The vessels are joined to form a double walled apparatus by use of an adapter part. When assembled, this double walled apparatus forms an air space between the inner and outer vessels, thereby providing insulation to promote temperature maintenance of liquids and/or solids held in the inner vessel. The air space can be filled with heated or cooled air or liquid, if desired. The vessel's insulating properties can be used to help warm, cool, and/or maintain the temperature of baby food.

In assembled form, this embodiment can be utilized at least for attachment to a breast pump for collection of expressed milk, and also as a feeding bottle via the application of an artificial nipple or other suitable attachment for feeding infants and small children.

To form this assembly, a vessel with male threads at its uppermost mouth end is preferably threaded into an adapter, forming a first assembly. The first assembly may be next inserted into a larger vessel. In so doing, the adapter of the first assembly is preferably press fit (or threaded, snapped, bayonet fastened, or inserted any other suitable way) into the larger vessel. The resulting apparatus becomes functional, such as when it is connected to a breast pump, in order to act as a collector of expressed milk, and can be engaged with a lid in order to act as a holding/storing container, or can be connected to an artificial nipple or other suitable attachment in order to act as a direct feeding vessel. It is preferable that connection of the apparatus to a pump, a cap or feeding attachment be by threaded or bayonet-type engagement.

Further, the outer bottle of this assembly can be designed so as to provide enhanced stability as a stand for the pump/vessel assembly when not in use. As the inner vessel is necessarily smaller than the outer vessel, and as the inner vessel is the container for the milk, the presence of the outer bottle can afford enhanced security for the held milk in tending to prevent spills.

Further, the outer bottle of this assembly provides protection from breakage of the inner vessel, which is the first holder of the milk.

Both vessels in the assembly can be produced using materials which provide sufficient clarity so as to enable the viewer to observe the milk in the inner vessel.

To utilize this assembly as a storage vessel for milk, a threaded cap or cap assembly can be engaged with the outside vessel for security of the contents. Similarly, the inner assembly may be removed, including the adapter, with an appropriate cap or appropriate feeding attachment then being secured to the inner bottle.

In another embodiment of this invention, two single walled bottles of compatible dimensions are molded with surface features which provide for ready interlocking of the two bottles, thereby forming an enclosed space between the two bottles. As with the first embodiment of this invention, the inner vessel holds fluid or solid food, with the outer vessel joined to form an air space for insulating properties. No adapter part is employed in this embodiment. As with the first embodiment, though, the assembly can be used in attachment to a breast pump as the collection vessel for expressed milk, and can readily be fitted with a cap or attachment for the feeding of held fluids.

In this second embodiment, a second, or inner, bottle is fitted into a first, or outer, bottle. The inner bottle is formed in such manner that its widest radial dimension is molded with convex and/or concave surfaces. These surfaces are preferably located near the top, or mouth end of the bottle. The outer or first bottle is formed in such manner that the narrowest radial area of its inner surface, preferably located in an area directly opposite a male threaded area on the outside of the first bottle, is molded with convex and/or concave surfaces designed to mate with complimentary convex and/or concave surfaces in the inner bottle in order for these two surfaces to easily accomplish the convenient interlocking of the inner and outer bottles, to form a reasonably secure insulating airspace. A lip or rim at the top or mouth end of the inner bottle has sufficient diameter so as to prevent the second bottle from entirely slipping into the inside area of the first bottle. The lip/rim of the second bottle, having been placed firmly in contact with the upper edge of the first bottle, also provides a degree of seal for the air space formed by the two bottles. The second, or inner, bottle further can have female threads, or the like, at its mouth end. When the second bottle is removed from the apparatus of this embodiment, a male threaded cap or plug or other suitable component can be engaged with the female threads or the like of this second bottle for purposes of sealing in the contents.

A third embodiment is similar to the second embodiment, without the interlocking elements. In this embodiment, the lip holds the inner bottle in place.

In this third embodiment, the apparatus may be used similarly for the direct expression of milk or for nipple feeding. This compatibility with expressing and feeding is optional, and is accomplished via male threads, or the like, located at the upper or mouth end of the first, or outer, vessel. Should it be preferable, each embodiment may or may not have threads on the outer vessel, and may simply act as a double walled apparatus for storing, holding, warming or enhancing temperature maintenance of milk, other liquids, or solids such as common baby food.

Reference will now be made, by way of example, to the accompanying drawings, in which:
FIG. 1 is a cut-away view of a first embodiment of a container made in accordance with the principles of this invention;
FIG. 2 is a perspective view of an insert used in the container of FIG. 1;
FIG. 3 is a cut-away view of the container of FIG. 1, with a closed cap on the container instead of a nipple;
FIG. 4 is a plan view of the container of FIG. 1, attached to a breast pump;
FIG. 5 is an exploded view of the container and breast pump of FIG. 4;
FIGs. 6(a) and 6(b) are cut-away views of a second embodiment of the present invention; and
FIG. 7 is a perspective view of a third embodiment of the present invention.

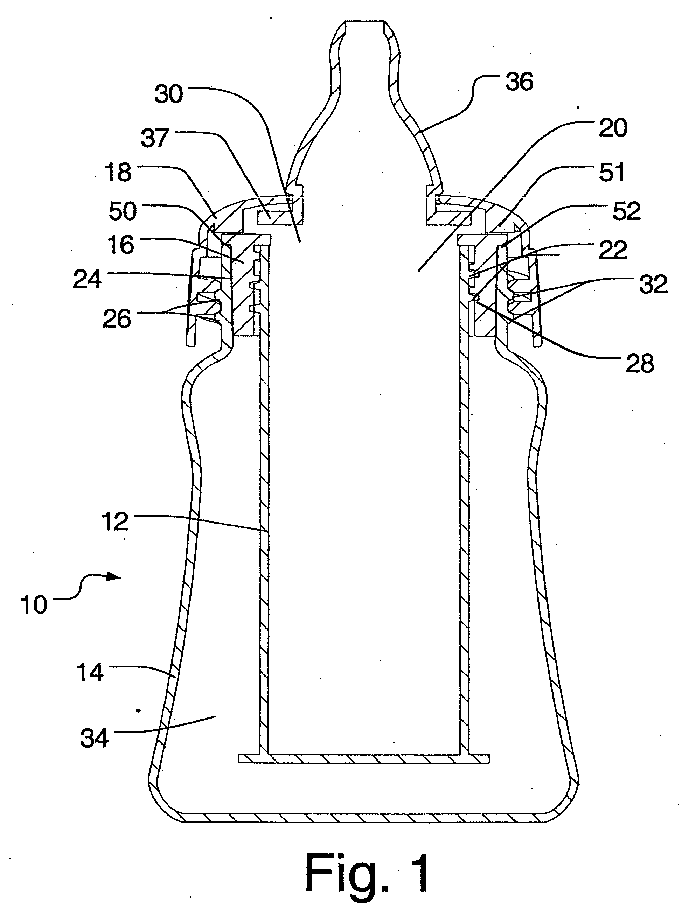

As seen in FIG. 1, a container 10 includes an inner vessel 12 for containing liquid or solid food, an outer vessel 14, an insert 16 and a cap 18. The inner vessel 12 has an open top end 20 and a plurality of threads 22 surrounding the outside of the top end 20.

The outer vessel 14 creates a larger volume than the volume of the inner vessel 12, so that the inner vessel 12 can fit inside the outer vessel 14. The outer vessel 14 also has an open top end 24, and a plurality of threads 26 surrounding the outside of the top end 24. The bottom of the outer vessel is larger than that of the inner vessel, providing a stable stand for the container when placed on the table or the like.

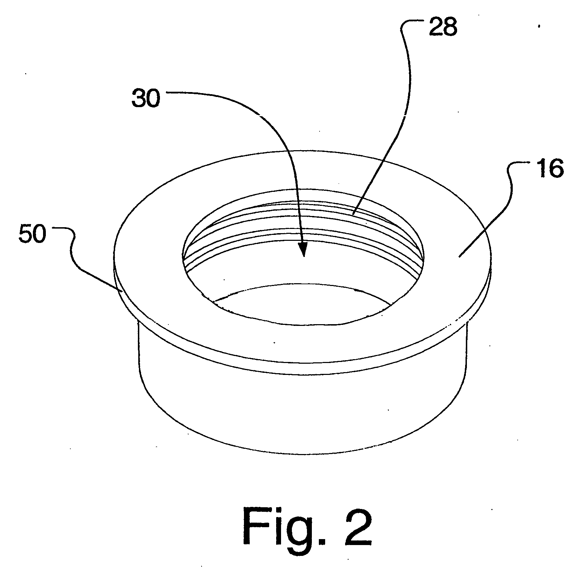

The insert 16 is located between the top end 20 of the inner vessel 12 and the top end 24 of the outer vessel 14. The insert 16 has a plurality of threads 28 (also seen in FIG. 2) that engage the threads of the inner vessel 12. The insert 16 has an opening 30. The insert 16 also has a lip 50 that extends over an upper edge 52 of the outer vessel 14. The insert 16 can be dimensioned such that typical commercially available baby bottles can be used for the vessel 12. The inner vessel 12 can also be a standard baby food jar.

The cap 18 (FIG. 1) covers the top ends of the first and second vessels, and the insert. The cap 18 has threads 32 that engage the threads 26 of the outer vessel 14.

Before the container is assembled, heated or cooled liquid (or warm or cool air) can be placed in the inner vessel 12, so that when the container is fully assembled, the liquid is in a cavity 34 between the first vessel 12 and the second vessel 14. The liquid is substantially sealed in the cavity by the insert 16, and the insert 16 is secured between the first and second vessels by the cap 18. A ridge 51 is provided to better place pressure against the insert 16 when the cap 18 is threaded and tightened.

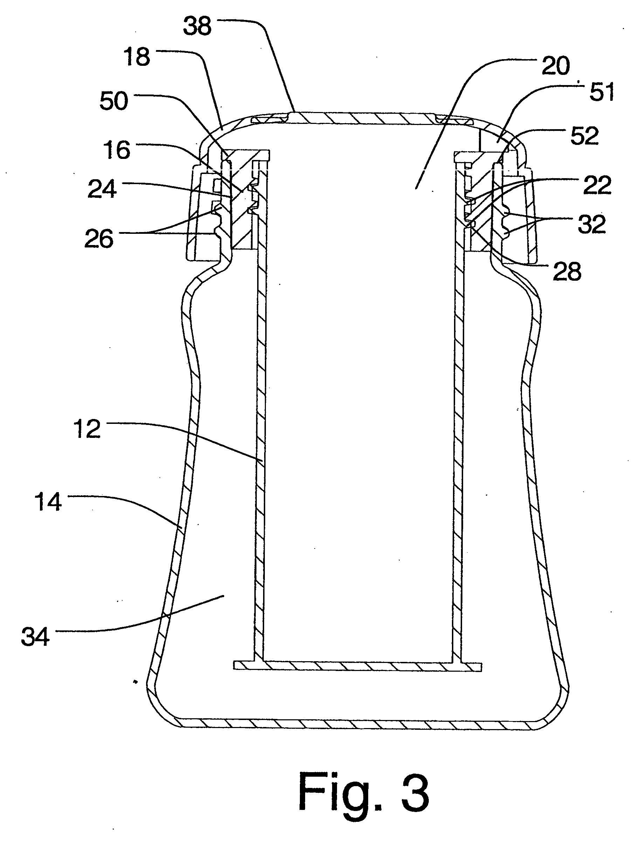

The cap 18 can have an opening for a nipple 36 having a base 37 as seen in FIGs. 1 and 2, or a cap 38 that is closed across its top can be used to seal the food in the inner vessel 12, as in FIG. 3. A cap with a sippee cup or any other suitable device could also be used, if desired.

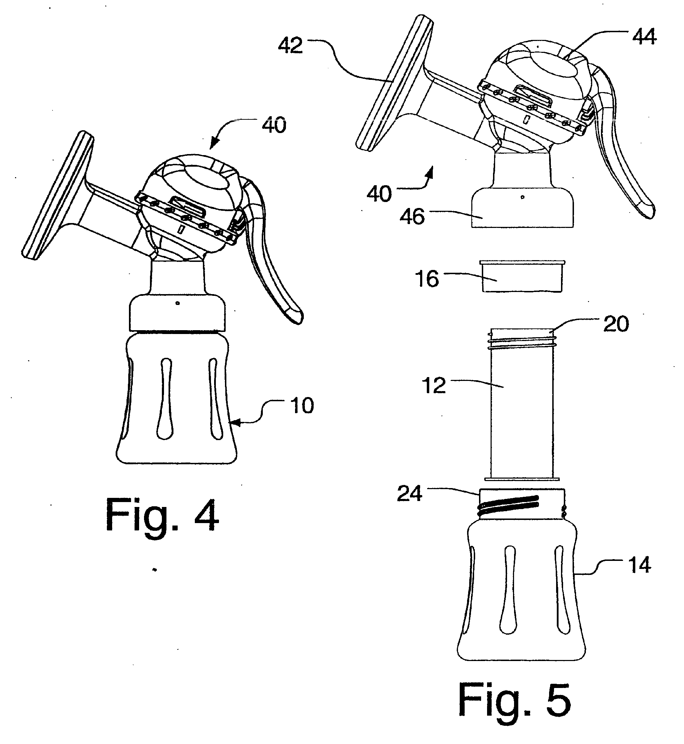

The container 10 can be secured to a manual breast pump 40, as seen in FIG. 4, or a powered breast pump, if preferred. The breast pump 40 includes a breast cup 42, a pump 44 and a manifold 46. The manifold 46 has an internally threaded opening (not seen in FIGs. 4 and 5) to which the container 10 may be easily secured.

The container 10 is assembled and secured to the breast pump 40 in the manner shown in FIG. 5. Air or liquid of a desired temperature is forced or placed in the outer vessel 14, and the inner vessel 12 is inserted through the open top end 24 of the vessel 14, after the adapter 16 is threaded to the top end 20 of the inner vessel 12. The manifold 46 is secured to the outer vessel 14.

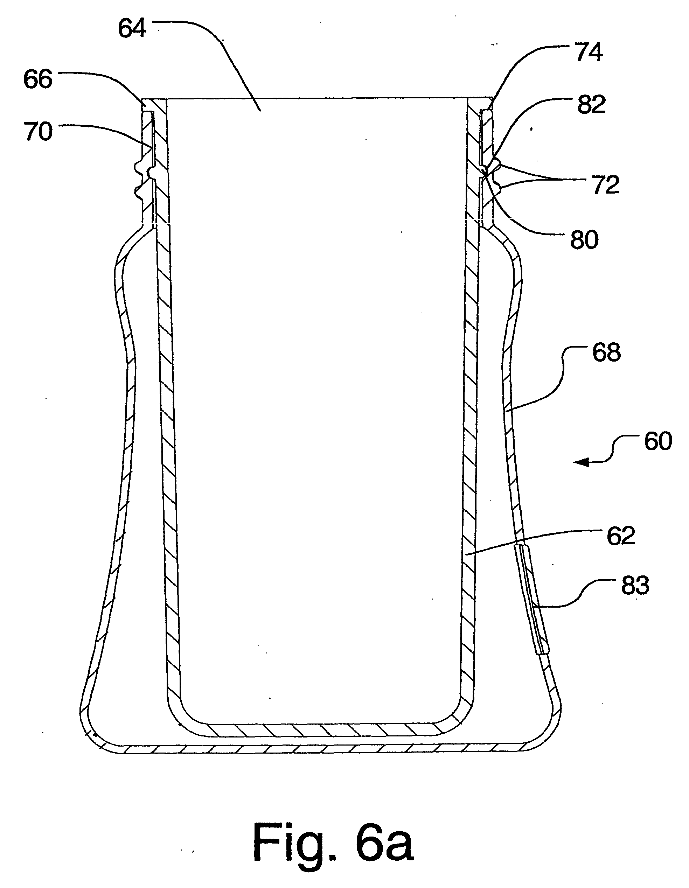

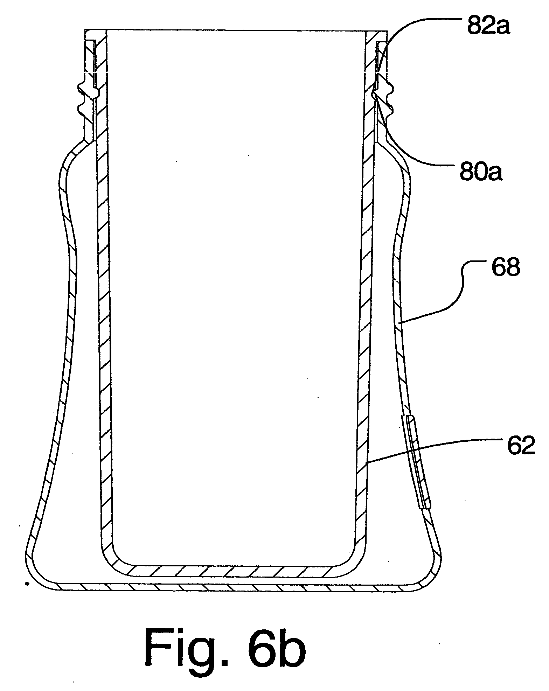

Another embodiment of the present invention is shown in FIGs. 6(a) and 6(b). A container 60 includes an inner vessel 62 that can hold liquid or solid food, such as milk. The inner vessel 62 has an open top end 64 and a lip 66 extending away from the top end 64 in the radial direction.

An outer vessel 68 has a top end 70 with threads 72 around the outside of the top end 70. The top end 70 also has a top edge 74.

The inner vessel 62 is dimensioned so that it fits within the outer vessel 68 and the top end 64 of the inner vessel 62 can be more or less press fit into the top end 70 of the outer vessel 68, possibly sealing the top ends of the vessels. The lip 66 engages the upper edge 74, to properly position the inner vessel 62 within the outer vessel 68, and seals the two vessels, as well. The embodiment of FIG. 6(a) also includes a concave protrusion 80 in the inner vessel 62 in a corresponding or mating convex indentation 82 in the outer vessel 68, to provide for releasable locking. FIG. 6(b) shows a variation of this embodiment, in which protrusions 80a are on the outer vessel 68, and indentations 82a are on the inner vessel 62.

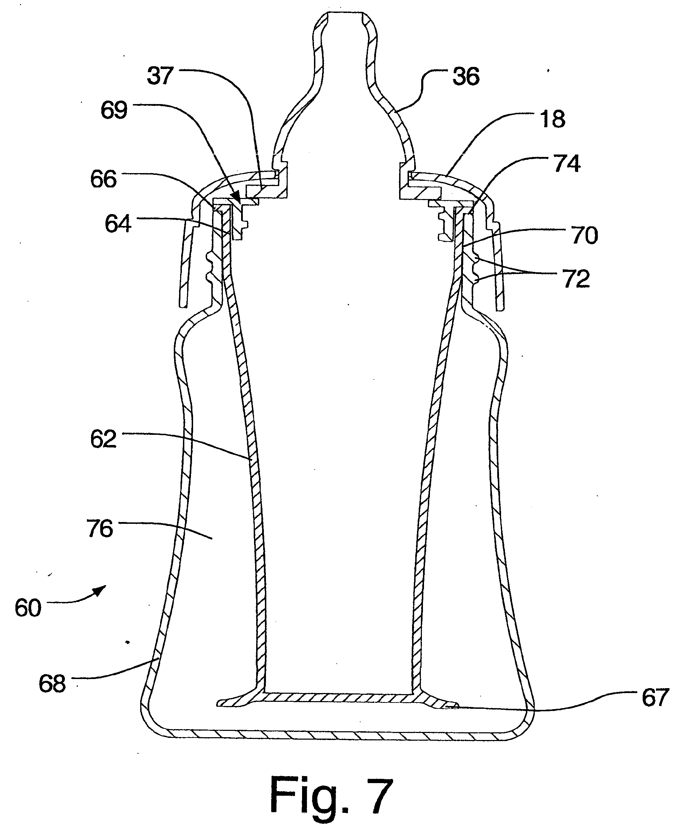

The releasable locking feature of FIGs. 6(a) and 6(b) is not used in the embodiment of FIG. 7, in which reference numerals from FIG. 6 are used where applicable. The inner vessel 62 of FIG. 7 also includes a flared bottom 67 that can be used to stand the vessel 62 upright when it is not inside an outer vessel 68.

A cap 18 and nipple 36 are shown in FIG. 7. A washer 69 is used because the base 37 of the nipple 36 does not reach the lip 74. The washer 69 can be similar to the insert 16 in FIG. 1. If the washer has the inner threads of the insert 16, the threads are simply not used in this embodiment. If the base of the nipple is sufficient to span the lip 74, then the nipple can be secured without a washer.

The inside threads of the cap 18 engage the outer threads 72 of the outer vessel 68, so that heated liquid or air may be placed in a cavity 76 formed between the inner vessel 62 and the outer vessel 68. The heated liquid or air may be substantially sealed in the cavity 78 by the press fit between the inner and outer vessels at their top ends and/or the lip 66 on the top edge 74 of the outer vessel, and the cap. As with the first embodiment, the cap can be closed at the top, or it can have a nipple, sippee cup spout or the like. It also can be secured to a breast pump, in the manner of the first embodiment. Attachment of the container 10 to breast pumps, artificial nipples or other common attachments, including caps for storage, can be by any appropriate means, such as bayonet twist, or various press fits.

The vessels are typically made of plastic, with enough flexibility so that the inner vessel can be inserted into the outer vessel and locked in place in a releasable manner so that it can also be easily removed, as desired. Clear material can be used as desired, for better visibility of contents.

Experiments using a two-ounce inner vessel revealed the good results obtained with the present invention. When the inner vessel was filled with liquid at body temperature (98.6° F), the temperature of the liquid without using this invention was 90° F after fifteen minutes, 85° F after thirty minutes, and 80° F after forty-five minutes. When the bottle was used in the present invention with air in the cavity, the temperature of the 98.6° F ingredients was 94° F after fifteen minutes, 90° F after thirty minutes, and 87° F after forty-five minutes. When the cavity was filled with 116° F water, which is similar to hot tap water in homes, the temperature of the 98.6° F ingredients rose to 102.5° F after fifteen minutes (a temperature favored by many lactation professionals), 96.4° F after thirty minutes, and 93° F after forty-five minutes. Thus, where the temperature of the contents of a conventional baby bottle without the present invention went from 98.6° F to 80° F in forty-five minutes, the contents of a bottle used in the present invention, with the cavity filled with hot tap water, started at 98.6° F and only dropped to 93° F after forty-five minutes.

The many advantages of this invention are now apparent. Baby bottles can readily be joined to form a double wall container for the purpose of better maintenance of desired temperatures as compared with temperature maintenance produced by the same bottles individually, and can be used to raise or lower the temperature of the food in the inner bottle by putting appropriate liquid (e.g., water) or air in the cavity between the inner and outer bottles. The containers are easy to assemble and disassemble for use and cleaning. Moreover, these containers allow users to realize extra value and economy through the inherent ability to readily provide a double walled, insulative vessel.

While the principles of the invention have been described above in connection with specific apparatus and applications, it is to be understood that this description is made only by way of example and not as a limitation on the scope of the invention.

## Claims

1. A container comprising:
an inner vessel for containing liquid or solid food, the inner vessel having an open top end and a plurality of threads surrounding the outside of the top end of the inner vessel;
an outer vessel that creates a larger volume than the volume of the inner vessel, so that the inner vessel can fit inside the outer vessel, the outer vessel also having an open top end and a plurality of threads surrounding the outside of the top end of the outer vessel;
an insert between the top end of the inner vessel and the top end of the outer vessel, the insert having a plurality of threads that engage the threads of the inner vessel, the insert having an opening; and
a cap that secures the insert and inner vessel in the outer vessel,
whereby liquid or air can be placed in a cavity formed between the first vessel and the second vessel, the liquid or air being substantially sealed by the insert, the insert being secured between the first and second vessels.

2. The container of claim 1, wherein the cap covers the top ends of the inner and outer vessels and the insert, the cap having threads that engage the threads of the outer vessel.

3. The container of claim 1 or 2, further comprising a nipple in the cap.

4. The container of any preceding claim, wherein the cap also seals the inner vessel.

5. A container for liquid or solid food comprising:
an inner vessel that can contain food, the inner vessel having an open top end and a lip extending away from the top end,
an outer vessel having an open top end and threads around the outside of the top end, the top end having an upper edge,
the inner vessel being dimensioned so that it fits within the outer vessel and the top end of the inner vessel fits into the top end of the outer vessel, the lip of the inner vessel engaging the upper edge of the outer vessel, and
a cap that secures the inner vessel in the outer vessel,
whereby liquid or air may be placed in a cavity formed between the inner and outer vessels, and the liquid or air may be substantially sealed in the cavity.

6. The container of claim 5, wherein the liquid or air is sealed in the cavity by a lip on the top edge of the outer vessel, and the cap.

7. The container of claim 5 or 6, wherein the liquid or air is sealed by a press fit between the inner and outer vessels.

8. The container of claim 5, 6 or 7, comprising means for releasably locking the inner vessel inside the outer vessel.

9. The container of claim 8, wherein the locking means includes a protrusion on the outside surface of the inner vessel, and a mating indentation on an inside surface of the outer vessel.

10. The container of claim 8, wherein the locking means includes a protrusion on the outside surface of the outer vessel, and a mating indentation on an inside surface of the inner vessel.

11. The container of any preceding claim, wherein the inner vessel is a baby bottle.

12. The container of any preceding claim, wherein the inner container is a baby food jar.

13. The container of any preceding claim, wherein the liquid or air in the cavity has an initial temperature higher than the initial temperature of the food in the inner vessel.

14. The container of any one of claims 1 to 12, wherein the liquid or air in the cavity has an initial temperature lower than the initial temperature of the food in the inner vessel.
